# EUROPEAN PATENT APPLICATION

(11) **EP 1 149 828 A1**
(43) Date of publication of application: **31.10.2001**
(21) Application number: 00201544.4
(22) Date of filing: 28.04.2000
(51) Int. Cl.: C07C 229/42, C07C 227/42

(54) **Crystalline form of diclofenac sodium salt**

(71) Applicant: AZIENDE CHIMICHE RIUNITE ANGELINI FRANCESCO A.C.R.A.F. S.p.A., 00181 Roma (IT)
(72) Inventor: Giannangeli, Marilena, 00181 Roma (IT)
(74) Representative: Marchi, Massimo, Dr.

(57) **Abstract**

Diclofenac sodium salt hemihydrate of formula (II) 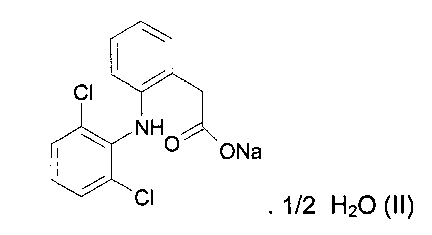 process for preparing thereof and pharmaceutical forms containing the same.

## Description

The present invention relates to a crystalline form of diclofenac sodium salt.

More particularly, the present invention relates to a crystalline form of diclofenac sodium salt which is not hygroscopic.

It is known that diclofenac sodium salt of formula (I) 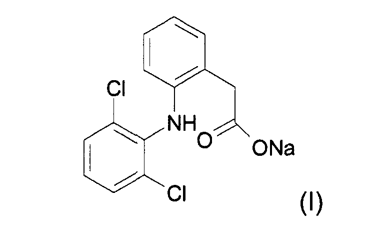 is a hygroscopic compound which melts at 283-285°C with decomposition.

Now, it has been surprisingly found that the crystalline hemihydrate form of diclofenac sodium salt of formula (II) 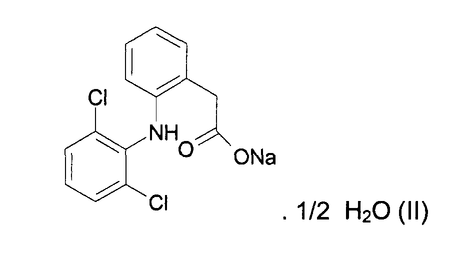 has no hygroscopic properties and this makes it particularly useful for preparing solid pharmaceutical forms such as powders, tablets and capsules.

Therefore, it is a first object of the present invention to provide diclofenac sodium salt hemihydrate of formula (II)

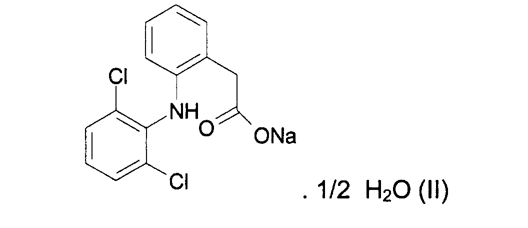

Further, it is a second object of the present invention to provide a method for obtaining diclofenac sodium salt hemihydrate of formula (II) 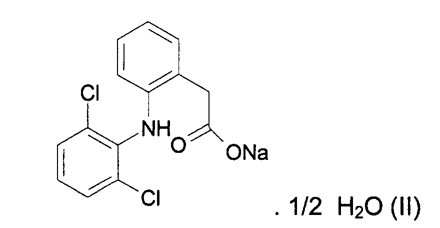 characterized in that diclofenac sodium salt of formula (I) 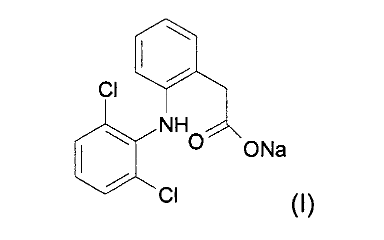 is crystallized from water in amounts equal to 2.5 parts by volume per one part by weight of diclofenac sodium salt.

It is a third object of the present invention to provide solid pharmaceutical forms containing, as active principle, diclofenac sodium salt hemihydrate of formula (II)

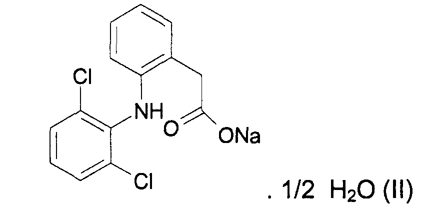

The example which follows is given to illustrate the present invention without, however, limiting it in any way.

### EXAMPLE

8 g of diclofenac sodium salt were crystallized from 20 ml of water.

Diclofenac sodium salt hemihydrate was thus obtained in almost quantitative yield (m.p. 286-288°C, with decomposition).

## Claims

1. Diclofenac sodium salt hemihydrate of formula (II)

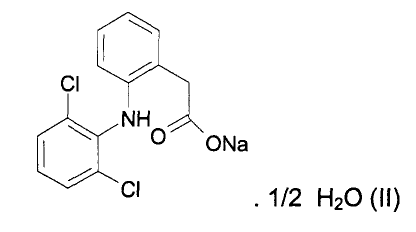

2. Method for obtaining diclofenac sodium salt hemihydrate of formula (II) 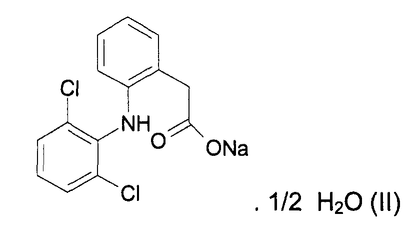 **characterized in that** diclofenac sodium salt of formula (I) 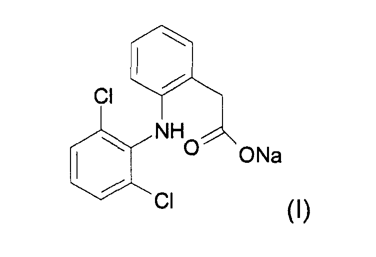 is crystallized from water in amounts equal to 2.5 parts by volume per one part by weight of diclofenac sodium salt.

3. Solid pharmaceutical form containing, as active principle, diclofenac sodium salt hemihydrate of formula (II)
